# EUROPEAN PATENT SPECIFICATION

(11) **EP 0 327 378 B1**
(45) Date of publication and mention of the grant of the patent: **11.12.1996**
(21) Application number: 89301052.0
(22) Date of filing: 03.02.1989
(51) Int. Cl.: C12N 15/00, C12P 21/00

(54) **Domain-modified constant region antibodies**
Modifikation der Domäne der Konstantregion der Antikörpern
Modification du domain de la region constant des anticorps

(30) Priority: 05.02.1988 US 152741
(43) Date of publication of application: 09.08.1989
(73) Proprietor: THE TRUSTEES OF COLUMBIA UNIVERSITY IN THE CITY OF NEW YORK, New York, New York 10027 (US); Becton Dickinson and Company, Franklin Lakes, New Jersey 07417-1880 (US)
(72) Inventor: Morrison,Sherie L, Scardsdale New York 10583 (US); Oi, Vernon T., Mount View, California 94040 (US)
(74) Representative: Harrison, David Christopher

(56) References cited:
- EP-A- 0 125 023
- EP-A- 0 184 187
- EP-A- 0 239 400
- WO-A-88/07089
- CHEMICAL ABSTRACTS vol. 107, no.7, 17th August 1987, abstract no. 57101r, Columbus, Ohio, US; MARC J. SCHULMAN et al.: & EUR. J. Immunol. 1987, vol. 17, no. 4, pages 549-554
- CHEMICAL ABSTRACTS vol. 100, no. 15, 9th April 1984, abstract no. 119073z, Columbus, Ohio, US; T. OI et al; & Miles Int. Symp. Ser. 1984, vol. 14, pages 281-287
- CHEMICAL ABSTRACTS vol. 105, no. 7, 18th August 1986, abstract no. 59187f, Columbus, Ohio, US; G. SARMAY et al; & Immunol. Lett. 1986, vol. 12, no. 5,6, pages 307-312
- PROC. NATL. ACAD. SCI. USA vol. 81, November 1984, pages 6851-6855, Washington, USA; SHERIE L. MORRISON et al; abstract, figure 1; page 6854c2
- NATURE vol. 312, 13th December 1984, pages 604-608, London, GB; MICHAEL S. NEUBERGER et al.: abstract, figure 1; page 605c2, page 607
- Oi and Morrison, BioTechniques, vol.4, no.3, pp.214-221 (1986)

## Description

The present invention relates to monoclonal antibodies and in particular to genetically modified monoclonal antibodies having domain-modified constant regions.

Naurally occurring specific binding molecules, such as immunoglobulins, enzymes, and membrane proteins, have seen an extraordinary expansion in commercial applications over the last decade. With the advent of monoclonal antibodies, the usefulness of immunoglobulins has been greatly expanded. However, in many applications, the use of monoclonal antibodies is severely restricted where the monoclonal antibodies are to be used in a biological environment. For example, monoclonal antibodies produced in rodents, e.g., mice, the most common producer species, are immunogenic to other species.

Cross-species immunogenicity is a function of the constant region of immunoglobulins. The constant region has a number of specific functions, such as complement binding, cell receptor binding control of catabolic rate, anaphylaxis, opsonization, placental and gut transfer, and immune regulation, in addition to being immunogenic. There will, therefore, be situations where it will be desirable to have constant regions which bind to cells or proteins from a particular species together with binding regions (variable regions) specific for a particular antigen.

Although it has generally been relatively easy to produce murine monoclonal antibodies of the desired antigen specificity, it has been much more difficult to produce human monoclonal antibodies with the desired constant region properties. Human monoclonals are preferable for many applications, especially in vivo diagnosis and therapy of humans. Chimeric antibodies with antigen specificity derived from a mouse myeloma cell or a hybridoma have been joined to human constant regions to overcome, in part, species limitations inherent in monoclonal antibodies. Genetically engineered antibodies produced by transfectomas have also allowed investigators to change the isotype of the antibody that results following fusion to a normal spleen cell, usually to produce IgG antibodies.

However, there have been obstacles encountered in using transfectomas to produce chimeric antibodies having murine variable regions and human constant regions. For example, initially-produced chimeric antibodies were limited to those in which the constant region had all of the properties of the donor human constant region. Enhanced or reduced properties, such as binding of complement, are desirable for many applications.

Accordingly, considerable interest remains in preparing immunoglobulins having modified biological functions while retaining the broad range of binding specificity available by use of variable regions from an easily available source.

### Relevant Literature

Oi et al., Proc. Natl. Acad. Sci. USA (1983) 80:825-829; Morrison et al., Proc. Natl. Acad. Sci. USA (1984) 81:6851-6855; Ochi et al., Proc. Natl. Acad. Sci. USA (1983) 80:6351-6355; and Ochi et al., Nature (London) (1983) 302:340-342 describe immunoglobulin expression in transfectomas. Neuberger et al., Nature (London) (1985) 314 :268-270 and Bouhanne et al., Nature (London) (1984) 312 :643 describe transfectomas secreting antibodies having mouse variable and human constant regions. Neuberger et al., Nature (London) (1984) 312:604-608 describe the expression of antigen binding domains covalently associated with non-immunoglobulin sequences. Advances in Immunology, Vol 40, pp. 61-134 reviews biologic activities residing in the Fc region of Ig. Immunoglobulin genes are disclosed and reviewed in numerous publications, such as Watson et al., Molecular Biology of the Gene, Vol. II, Benjamin/Cummings, Menlo Park, California, 4th edition, 1987, Chapter 23, and the publications cited therein.

EP-A-125023 discloses antibody chains in which an entire constant region of one type has been substituted by a constant region of another type and EP-A-184187 discloses the preparation of chimeric immunoglobulin gene expression plasmids which contain DNA encoding an entire mouse immunoglobulin H-chain variable region ligated to a DNA fragment encoding an entire human constant region.

In contrast, in the invention an antibody having at least one binding site region and a domain-modified constant region is provided in which the domain modification is either a substitution of, an insertion of, or a deletion of substantially all of the amino acids of at least one of the domains of a constant region: C_{L}, C_{H}1, hinge, C_{H}2, C_{H}3 or C_{H}4. The functional properties of the domain-modified constant region antibodies are selected to enhance the desired biological functions for a particular application. Antibody constant-region domains can be eliminated, inserted, or substituted for (exchanged) by a domain of a different isotype or immunoglobulin class or from the light chain. The substituted or inserted domain can be from the same antibody or from another antibody of the same animal, the same species of animal, or a different species of animal. In this way, the functional properties of the biological molecule can be optimized for the desired application.

DNA constructs encoding domain-modified constant regions, constructs encoding complete constant-region-domain-modified antibodies, and cells expressing such antibodies are also provided.

### BRIEF DESCRIPTION OF THE DRAWINGS

Figure 1 illustrates a cloning cassette used to exchange constant regions;
Figure 2 illustrates a cloning cassette used to exchange constant region domains;
Figure 3 illustrates a cloning cassette used to delete or duplicate constant region domains;
Figure 4 illustrates a cloning cassette used to delete a carboxy terminal domain; and
Figure 5 illustrates two variations of cloning cassettes used to delete an amino terminal domain.

### DESCRIPTION OF THE SPECIFIC EMBODIMENTS

Novel methods and compositions are provided for production of antibodies having domain-modified constant regions. The present invention is concerned specifically with modifications of one or more domains of the constant region of an antibody, which can be or is attached to a binding-site or variable region of the original antibody from which the constant region was derived or to another antibody. The complete antibodies have at least one binding site region which may be naturally occurring in a host animal or genetically modified. Alterations of the binding site region of the antibodies do not constitute a part of this invention, although such altered-binding-site antibodies will be within the scope of this invention if their constant regions are modified as described herein.

As used herein, a domain-modified constant region means that substantially all of the amino acids of at least one of the domains of C_{L}, C_{H}1, hinge, C_{H}2, C_{H}3 or C_{H}4 in an antibody chain is either deleted, inserted or exchanged. By exchanged is meant that a domain from a different source is substituted for a domain initially present in the antibody. When the modification is a substitution, usually the domain is substituted for by the equivalent domain of an antibody of another isotype or class or from the light chain of the same or a different antibody. Usually the insertion of a domain is the duplication of one or more domains of the original antibody. However, other substitutions and/or duplications can also be carried out.

Antibodies having a domain-modified constant region can be prepared from fused genes containing a gene segment encoding a portion of the antibody involved with binding and a gene segment encoding a domain-modified constant region derived from the same or a different host animal. Two expression cassettes, one encoding the heavy chain and one encoding the light chain, are generally involved in producing complete domain-modified constant region antibodies, which genes are introduced into an appropriate eukaryotic host under conditions for expression and processing. When the genes are expressed and the antibody chains bind together, an assembled antibody is obtained. The antibodies include IgM, IgG, IgA, IgD and IgE classes as well as the various subclasses of the individual classes (e.g, human IgGl through IgG4). Chain types include for the light chain κ or λ and for the heavy chain µ, γ, α, δ and ε. Classification by isotype refers to the class and subclass, light chain type and subtype, and can also be applied to the variable region groups and subgroups. An example of an isotype designation for a mouse immunoglobulin is IgG₂ₐ(κ).

The binding site or variable region will vary in conformation with the amino acid sequence providing the desired specificity. The variable region of the immunoglobulins will be derived from a convenient source, usually mammalian, which may be a rodent, e.g., mouse or rat, rabbit, or other vertebrate, mammalian or otherwise, capable of producing immunoglobulins. The variable region may be genetically modified or naturally occurring.

The constant region of the immunoglobulin, as well as the J chain for IgM (not the same as the J region of the heavy or light immunoglobulin chain), will be derived from a vertebrate source the same as or different from the source of the variable region, particularly a mammalian source, more particularly primate or domestic animal, e.g., bovine, porcine, equine, canine, feline, or the like, and most particularly human. The source of the constant region can be either from the same animal or different animals of the same or different species as the source of the variable region.

As is known, the constant region of an antibody chain is specific for a species. Within each class there will be a plurality of allotypes. The domain-modified constant-region portion of the antibody will normally be chosen in accordance with the intended uses of the antibody. As a first consideration where the antibody is to be introduced into a host animal for diagnosis or therapy, the constant or immunogenic portion will be selected so as to minimize the immune response of the host. Thus frequently, the source of the constant region is human when intended for use in humans.

The potential application of antibody molecules are determined not only by their antigen specificity but also by their effector functions. Therefore, as a second consideration, the domain-modified antibodies are selected to optimize their ability to perform a particular function. For example, the domain or domains related to enhancing the half-life of the antibodies will be modified to provide an enhanced or shortened half-life depending on the particular application. Other functions that can be selectively modified in antibodies using the techniques taught herein include tissue distribution, ability to fix complement, and ability to participate in antibody-dependent cellular cytotoxicity.

As a third consideration, the antibodies are modified to facilitate in vitro manipulations. For example, antibodies can be altered so that it is easier to add more molecules of a radioactive isotope or drug without changing the binding site specificity. If the antibody is to function as a drug delivery system, enhancing covalent attachment of the drug in an antibody that can be secreted in large quantities would be desirable.

The modification of the constant region can be the deletion, insertion, or substitution of substantially all of the amino acids of at least one domain. "Substantially all," when referring to the amino acids of a domain, encompasses 100% of the amino acids being manipulated but can also refer to less than 100% of the amino acids, such as 80%, 85%, 90%, or 95%. Manipulation of 100% of the amino acids in a domain by use of restriction sites in introns is most common. A number of antibody functions have been localized to a domain or domains as illustrated in Table 1. See Paul, Fundamental Immunology, Raven Press, New York, NY, 1984.

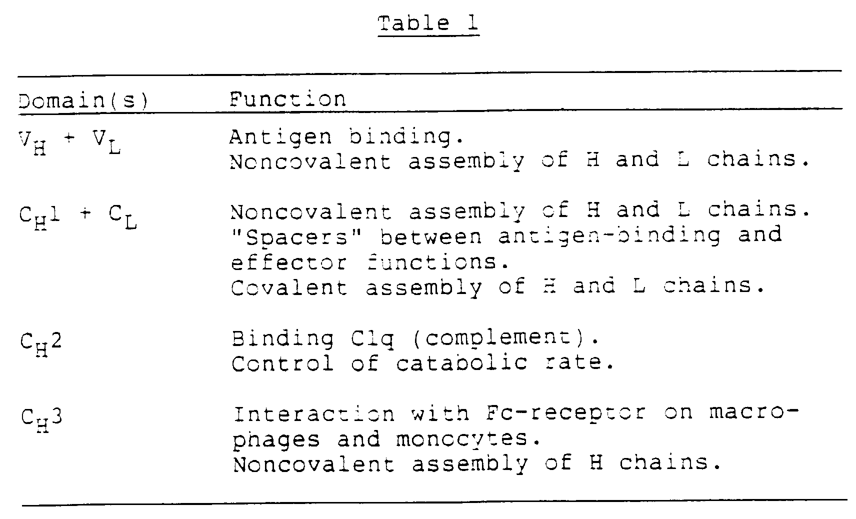

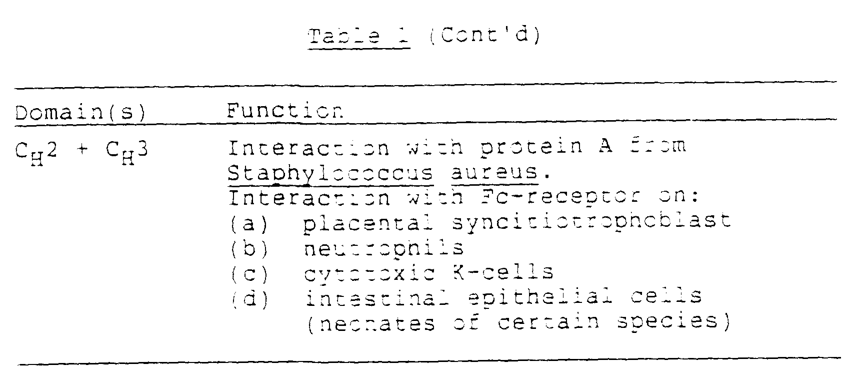

Since the function of these domains is known, a particular effector function can be modified by deleting or inserting a domain related to that function or by substituting a domain of a different class or isotype antibody for a corresponding domain which functions less effectively for a desired property.

One or more deletion can occur in preparing a domain-modified constant region of the invention. Multiple deleted domains may be adjacent or non-adjacent. The deleted domain(s) can be the carboxy-terminal domain of the constant region, one or more internal domains, or the amino-terminal domain. Domains related to functions not necessary for or detrimental to a particular use can be eliminated. For example, the C_{H}2 domain can be deleted when complement binding is undesirable. Deletion of non-essential domains may facilitate secretion or assembly of antibody molecules. For example, deletion of C_{H}1, the proposed binding site of heavy chain binding protein, would permit secretion of heavy chains without associated light chains. Domain deletions can be selected to influence any of the biologic properties of immunoglobulin.

When the constant region of a heavy chain is modified by a substitution, the substitution involves substantially all of the amino acids of at least one of the domains of C_{L}, C_{H}1, hinge, C_{H}2 or C_{H}3, or in the case of IgM and IgE, the C_{H}4 domain. The domain can be substituted for with a constant region domain from an antibody of another isotype or class. Additionally, the light chain constant domain can be substituted for heavy region domains, including joining the heavy chain variable region (V_{H}) to the light chain constant region (C_{L}). Usually, but not necessarily, a domain will be substituted for by the corresponding domain of an antibody of another isotype or class. That is, for example, a C_{H}1 domain will usually be exchanged for another C_{H}1 domain.

In addition to exchanging domains in order to produce antibodies having currently predictable properties, molecules with unique combinations of biological functions not now known can be prepared as greater insight is gained into the contribution of each domain to biologic activity. For example, it should be possible to combine the ability of IgG₃ to bind Fc receptors on mononuclear cells with the longer serum half-life of IgG₂ if these properties are associated with different domains. The reduced ability of IgG₄ to activate complement may be combined with the ability of IgG₁ to bind with high affinty to Fc receptors. By combining domains from IgA and IgG, it may be possible to produce IgG molecules that can interact with a secretory piece and so be less susceptible to proteolysis in the gut.

The constant region can also be domain-modified by the insertion of substantially all of the amino acids of at least one of the domains of the constant region, C_{L}, C_{H}1, C_{H}2, C_{H}3 or C_{H}4. In some cases the insertion provides a duplicate of at least one constant region domain already present to provide a constant region having one or more of the domains present as multiple copies. Additionally, the inserted domain can be from an antibody of a different isotype or class or from another chain. Usually the insertion will maintain the domains in their natural order. That is, for example, all hinge domains present will be between the C_{H}1 and C_{H}2 domain regions. Alternatively, when two or more adjacent domains are inserted, the natural order may be maintained within the inserted group. That is, a second copy of the C_{H}1 and hinge domains may follow the first hinge domain. Domain duplication can also be used to enhance desired functions. For example, carbohydrate is contained in the C_{H}2 domain of IgG; carbohydrate has been used for attaching radioactive labels to Ig. By duplicating C_{H}2, more carbohydrate will be present and potentially available for use in labeling techniques.

The substituted or inserted domain can be from the same host animal source as the constant region or from a different source. The different source may be from the same or a different species. The substituted or inserted domain can also be a naturally occuring or a genetically modified domain. Genetic modifications contemplated include the addition of amino acids facilitating covalent linking of drugs or radioisotopes, such as by including cysteines or lysines in the domain, usually as a substitution for an amino acid present in the domain as obtained from natural sources. Another anticipated genetic modification would be addition or deletion of carbohydrate, which could be accomplished by substitution of amino acids to provide additional amino acids or delete amino acids that serve as points of attachment for carbohydrates.

A number of exemplary domain-modified constant region antibodies which have been produced are listed in Table 2 in the experimental section. In addition to those listed, a series of γ₃ proteins with 0, 1, 2, 3, 4, and 7 hinge exons have been produced. Proteins in which V_{H} has been joined to C_{L} and C_{L} to V_{H} have also been produced. These proteins bound antigen and were secreted.

Antibodies of the invention are generally produced in microorganisms or cell lines using an expression vector system comprising transcriptional and translational regulatory sequences and a gene capable of producing a domain-modified constant region under the control of the regulatory sequences. Suitable expression systems are well known and do not constitute a part of this invention. Vectors used for transfection of Ig genes are described in Bio Techniques 4:214-221. These vectors provide one feasible delivery system; delivery by alternative vectors would also be within the scope of the present invention.

The general expression vector systems employed to produce the antibodies of the present invention can be varied in order to provide a number of convenient properties. First, the H and L chains can be on separate plasmids. Although both genes may be on the same plasmid and there is no inherent size limitation, it is more convenient to genetically manipulate smaller plasmids due to the number of novel restriction sites available. Second, unique sites can be introduced within the vectors using linkers to facilitate transferring variable regions between plasmids. This is more easily accomplished in smaller plasmids. Third, once a variable region has been cloned into one expression vector, it is simpler to express it associated with different constant regions in a small plasmid than to require two changes in the same large plasmid prior to expression.

The gene segments encoding the individual antibody domains are typically joined by linking groups. These linking groups can be natural introns or can be introns that have been modified by artificial manipulation. The intron/exon nature of the antibody gene can be used to facilitate preparation of domain-modified constant regions of the invention. The domain structure of antibody molecules is reflected in the structure of the genes which encode it, with each domain being encoded by a separate exon. The intervening DNA sequences (introns) separating each domain provided ideal sites for manipulating the antibody gene. Since the intervening sequences are removed by RNA splicing and are not found in the mature mRNA, alterations within the intervening sequence do not affect the structure of the mature protein molecule.

In addition, the RNA splice junctions between variable and constant region exons in both heavy and light chain genes can be used to advantage in the present invention since these junctions are conserved, even between species. The amino acid at the domain boundary is always formed by two nucleotides from the 5′ domain and one nucleotide from the 3′ domain. Thus by ligating gene segments within the intervening DNA sequences, it is possible to make chimeric molecules within and between chains as well as across species.

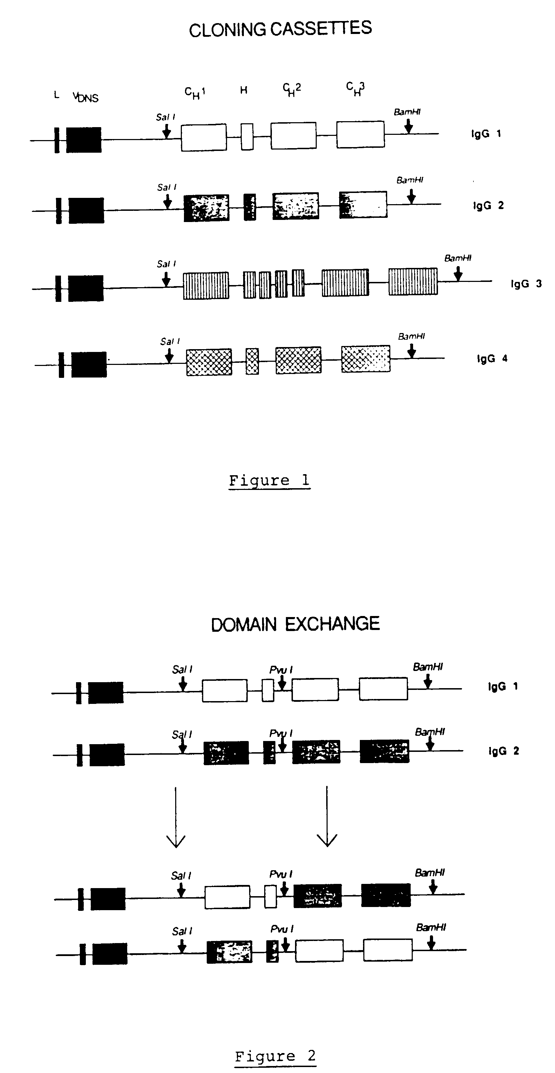

The general strategy typically used for making chimeric molecules of the invention is to create cloning cassettes (see Figure 1) containing at least the domains to be manipulated. A similar technique has been used in the prior art for preparing chimeric mouse variable/human constant region heavy chains. When the vectors for creating chimeric mouse variable/human constant region heavy chains are constructed, for example, a SalI site can be introduced into the intervening sequence using DNA oligonucleotide linkers. This renders the human constant region gene segments into SalI-BamHI fragments and facilitates the insertion of different constant regions next to the variable region gene segments. Once a variable region prepared in this fashion is cloned, the variable region can be placed next to different constant regions in new cassettes.

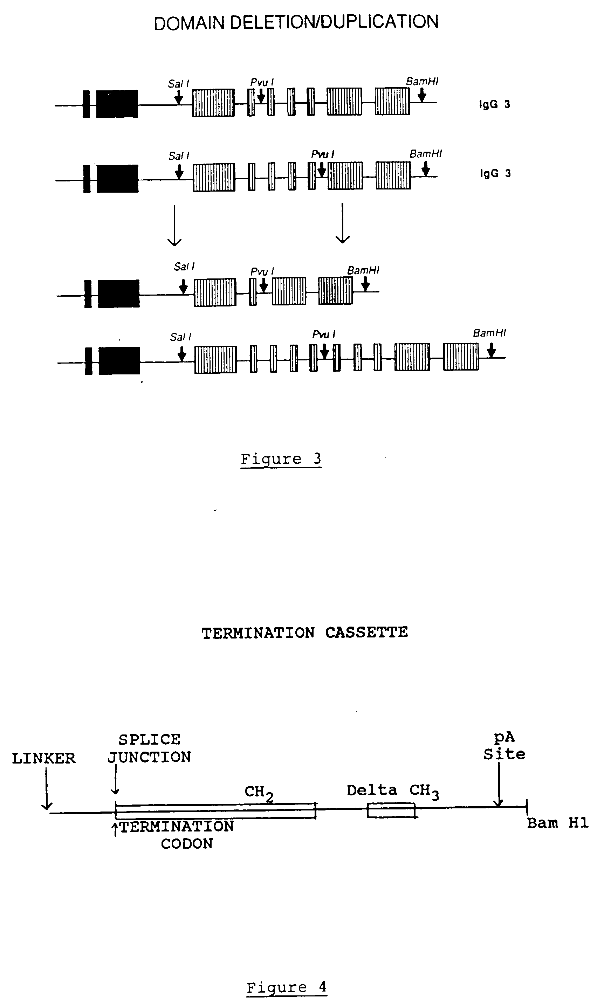

It has now been determined that a similar approach can be used to create domain cassettes for use in preparing domain-modified antibody chains (see Figure 2). Using oligonucleotide linkers, unique restriction sites can be introduced into one or more of the intervening sequences separating the exons that encode the domains of the constant-region IgG heavy-chain genes. Examples of restriction sites are set forth in the Figures. Once these linkers are in place, it is possible to exchange, insert, or delete exons and hence immunoglobulin domains (see Figure 3). The new vectors produced by exchange, insertion, or deletion can then be used to produce immunoglobulin molecules having altered structures which enhance the desired functional property.

Specific techniques are described in the examples that follow. Furthermore, now that the present invention has demonstrated that restriction sites can be introduced into the intervening sequences separating the exons that encode the domains, as well a between constant and variable regions, the techniques described in various scientific publications relating to the production of chimeric antibody molecules with antigen-binding domains and constant region domains from different mammalian sources can be applied to the exchange, insertions, and deletion of domains within the constant region using the guidance provided in this specification. A number of relevant publications are set forth above in the section entitled Relevant Literature.

The above strategy is also effective for producing internal deletions within the Ig molecule. Slightly different approaches can be used to create deletions at either the amino or carboxy terminus of the molecule.

There are two ways to make a carboxy terminal deletion. The first is to insert a non-sense codon by site-directed mutagenesis. Using this approach chains terminating at any residue can be created. As an alternative approach, a cassette can be produced and placed 3′ of an exon to cause termination after that exon. As starting material, the gene encoding, for example, C_{H}2b-C_{H}3 of mouse γ_{2b} can be used. This gene contains convenient restriction sites. First, most of C_{H}3 is deleted. Then a termination codon in all three reading frames is inserted at the 5′ end of the C_{H}2 domain. A unique linker can be inserted into the IVS 5′ of C_{H}2. This cassette can then be placed 3′ of any exon. The preceding exon can be spliced to C_{H}2 of γ_{2b}; the resulting message contains a termination codon at this point.

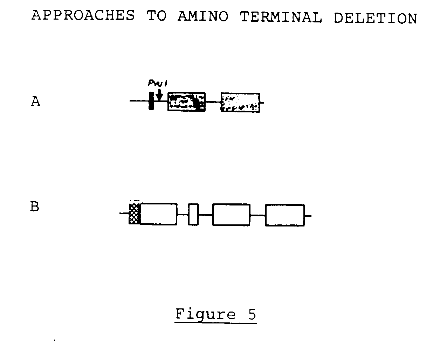

Several approaches to making amino terminal deletions are possible, and two such approaches are illustrated in Figure 5. In Figure 5A the 5′ region of the Ig gene with its promoter and leader are joined to the exons to be expressed. The leader is spliced in reading frame to the exon 3′ of it, producing a mRNA with appropriate start sites and encoding a protein. The leader sequence may or may not be cleaved from the completed protein. In Figure 5B the promoter regions and other regions necessary for Ig transcription (indicated by cross-hatched box) are joined directly to the exon. If a translation start site (AUG) is not available, it can be provided by in vitro mutagenesis. The position of the translation start site will determine the size and structure of the protein product.

The invention also contemplates DNA constructs for producing a domain-modified constant region. The constructs comprise DNA sequences encoding polypeptide segments substantially the same as each of the constant region domains joined through a linking group in reading frame at its 5′ end to the 3′ end of the sequence encoding the previous domain. The constructs will usually additionally include a sequence encoding the variable region, joined through a linking group at its 3′ end to the 5′ end of the sequence encoding the first constant region domain. When the construct encodes the heavy chain it will include sequences encoding the C_{H}1, hinge, C_{H}2, C_{H}3 and optionally the C_{H}4 domains. Similarly, the construct will contain sequences encoding the C_{L} domain when the construct encodes the light chain. The constructs can be synthesized in segments that are spliced together and cloned, or by any other technique of biotechnology.

When the domain modification is a substitution, the linking group preceding and following the gene segment(s) encoding the domain(s) to be exchanged will usually comprise a unique restriction site. The same restriction site then will be present in the linking groups surrounding the sequence encoding the domain(s) of another antibody which is to be exchanged. To facilitate duplications or internal deletions, the same first unique restriction site is present in the linking group which precedes the sequence encoding the domain which is at the amino terminal end of the sequence encoding the domain to be duplicated or deleted. A second unique restriction is present in the linking group preceding the sequence encoding the domain(s) to be duplicated/deleted in the gene encoding one chain and in the linking group following the sequence encoding the domain(s) in the gene encoding a second identical chain. In this way, following exchange, the domain(s) is present in two copies (duplicated) in the gene encoding one chain and absent (deleted) in the gene encoding the second chain. When the domain modification is a carboxy-terminal deletion, the linking group following the domain which will comprise the C-terminus will contain either a non-sense codon or a termination codon.

Cells producing antibodies of the present invention are also provided. The cells contain DNA constructs for expression of a domain-modified constant region antibody heavy chain described previously. The cells also contain a DNA sequence encoding a light chain region, usually in a separate DNA construct. The production of cells producing chimeric antibodies (e.g., transfectomas) is well known and described in detail in articles cited in Relevant Literature.

In addition to complete antibodies, antibody fragments containing domain-modified constant regions also are a part of the present invention. For example, half antibodies prepared from single light and single heavy chains that have assembled to form a specific binding site can be used as monovalent binding proteins. In addition, non-assembled domain-modified constant regions can be used as antigens for generating specific polyclonal or monoclonal antibodies. Production of particular antibody specificity can be eliminated or enhanced by deletions or insertions, respectively. Use of a domain-modified antibody having exchanged domains can produce polyclonal antibodies having a unique mixture of specificities in high yield.

The term "antibody" as used in this application, refers to both whole antibodies and fragments thereof. Whole antibodies contain two light and two heavy chains assembled into the natural Y-shape configuration. Antibody fragments include half antibodies formed from single light and heavy chains that have assembled to produce a specific binding site as well as fragments of either whole or half antibodies prepared by cleavage with enzymes, such as papain.

The following examples are offered by way of illustration and not by way of limitation.

### EXPERIMENTAL

### Example 1

Human γ₃ antibody has an extended hinge region consisting of four exons. Human γ₄ exhibits quite different properties in spite of extensive sequence similarities (>90% identical residues in C_{H}2 and C_{H}3) in their constant regions. IgG₃ genes have been constructed and expressed. The proteins isolated have one, two, three, or four (wild-type) hinge exons. A change in molecular weight of the heavy chains with the subsequent hinge deletions was noted. In addition, the γ₃ hinge has been placed in the γ₄ constant region and vice versa. Exchanging the hinge region from IgG₃ and IgG₄ did not alter the ability of these molecules to fix complement and to bind to the Fc receptor.

### Example 2

In order to maximize the usefulness of genetically engineered antibodies for such applications as drug delivery, it is useful to make alterations in the antibody. The limitations are that the antibodies must be assembled and secreted and must retain their ability to specifically bind antigen.

To determine if there is an upper limit on the size of an antibody molecule which could be produced, an IgG₃ heavy chain in which C_{H}1 and the hinge region were duplicated was constructed. In a second construct, C_{H}1, hinge and C_{H}2 were duplicated. When the gene with C_{H}1 and the hinge duplicated was used for transfection a greatly reduced transfection frequency was seen. When the surviving transfectants were analyzed, none produced any detectable heavy chain protein. These results suggest that the heavy chain encoded by this construct was toxic to the cells.

In constrast, the gene encoding a heavy chain in which C_{H}1, hinge, and C_{H}2 were duplicated was expressed following transfection. The chain assembled with a mouse λ light chain and was secreted. These studies suggest that while there will be limitations as to specific Ig molecules which can be produced, these limitations are not inherently size related.

### Example 3

In a related series of studies, deletions of various domains that affect the ability of Ig molecules to assemble, to be secreted, and to function were made. IgG₃ heavy chain genes encoding proteins with deletions of
C_{H}2;
hinge + C_{H}2;
C_{H}1 + hinge; and
C_{H}1 + hinge + C_{H}2
were constructed and transfected into myeloma cells.

Chimeric heavy chains in which a domain(s) has been deleted were synthesized. J558L was transfected with the chimeric heavy chain gene and transfectants synthesizing protein were isolated. Transfectants were labeled for 3 hours using ³⁵S-methionine. Cytoplasmic and secreted Ig were prepared by immunoprecipitating with anti-heavy chain. The immunoprecipitates were analyzed by SDS-PAGE.

When the gene with the deletion of C_{H}2 was used, it was found to direct the synthesis of a protein of the expected molecular weight. This shortened heavy chain assembled with either mouse λ light chain or chimeric V-DNS-human κ light chain into an H₂L₂ molecule which was secreted.

When the hinge + C_{H}2 were deleted, the heavy chain apparently assembled into HL half molecules which were secreted. However, it is impossible to say conclusively that these are not H₂.

When C_{H}1 + the hinge were deleted, no assembly of the heavy chain with either other heavy chains or with L chain occurred. This is not surprising since the free cysteine which forms the interchain disulfide bonds is present in C_{H}1. However, even in the absence of interchain disulfide bonds, the shortened heavy chain is secreted.

A heavy chain in which the variable region is directly joined to C_{H}3 was also produced. This heavy chain was secreted when cotransfected with a chimeric light chain into a non-producing myeloma. It did not form covalent bonds with either light chain or another heavy chain.

### Example 4

Table 2 illustrates a number of domain-modified constant region antibodies which have been produced.

**Table 2**

| Domain Exchange Proteins Which Have Been Produced | | | | |
|---|---|---|---|---|
| Vector Number | C_{H}1 | Hinge | C_{H}2 | C_{H}3 |
| 1658 | γ₂ | γ₃ | γ₃ | γ₃ |
| 1647 | γ₃ | γ₂ | γ₂ | γ₂ |
| 1654 | γ₃ | γ₃ | γ₂ | γ₂ |
| 1656 | γ₂ | γ₂ | γ₂ | γ₃ |
| 1657 | γ₃ | γ₃ | γ₃ | γ₂ |
| 1673 | γ₁ | γ₁ | γ₄ | γ₄ |
| 1672 | γ₄ | γ₁ | γ₁ | γ₁ |
| 1227 | γ₃ | γ₄ | γ₄ | γ₄ |
| 1228 | γ₄ | γ₃ | γ₃ | γ₃ |

Production of proteins prepared from the vectors set forth in Table 2 and analysis of their functions has begun. For example, vector 1654 produces a protein that has been analyzed for its Fc receptor binding activity and, like IgG₂, does not bind to the Fc receptor of human monocyte cell lines.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto.

## Claims

1. An antibody chain having at least one binding site region and a domain-modified constant region, wherein said domain-modified constant region comprises a modification selected from the group consisting of (1) an insertion of at least eighty percent of the amino acids of at least one of the domains of C_{L}, C_{H}1, hinge, C_{H}2, C_{H}3, and C_{H}4, wherein the rest of the domain-modified constant region has the same amino acid sequence as that of a constant region of a mammalian antibody, (2) a substitution of at least eighty percent of the amino acids of at least one of the domains of C_{L}, C_{H}1, hinge, C_{H}2, C_{H}3, or C_{H}4 by at least eighty percent of the amino acids of at least one but less than all of said domains from a different mammalian antibody chain, wherein the rest of the domain-modified constant region has at least eighty percent the same amino acid sequence as that of a constant region of a mammalian antibody and (3) a deletion of at least eighty percent of the amino acids of more than one of the domains C_{H}1, hinge, C_{H}2, C_{H}3, or C_{H}4, wherein the remaining amino acids of the domain-modified constant region have at least eighty percent the same amino acid sequence as that of at least one domain of a constant region of a mammalian antibody.

2. An antibody chain of Claim 1, wherein said binding site region and said domain-modified constant region are from the same mammalian source.

3. An antibody chain of Claim 1, wherein said binding site region is from a mammalian first source and said domain-modified constant region is from a mammalian second source.

4. An antibody chain of Claim 3, wherein said mammalian first source is of the same species as said mammalian second source.

5. An antibody chain of Claim 3, wherein said mammalian first source is of a different species from said mammalian second source.

6. An antibody chain of Claim 1, wherein said domain-modified constant region comprises a substitution of a corresponding domain of an antibody of another isotype or class.

7. An antibody chain of Claim 1, wherein said substituted domain is from the same species of animal as said constant region.

8. An antibody chain of Claim 1, wherein the source of said substituted domain is from a different species than the source of said constant region.

9. An antibody chain of Claim 1, wherein said insertion is of at least two domains.

10. An antibody chain of Claim 1, wherein said insertion is the duplication of at least one domain of said constant region.

11. An antibody comprising at least one antibody chain of Claim 1.

12. A DNA construct, comprising a DNA sequence encoding an antibody chain of Claim 1.

13. A DNA construct for producing a domain-modified constant region of an antibody heavy chain comprising:
(a) a first DNA sequence coding for a polypeptide at least eighty percent the same as the C_{H}1 region of an antibody heavy chain;
(b) a second DNA sequence joined through a linking group in reading frame at its 5' end to the 3' end of said first DNA sequence, said second sequence coding for a polypeptide at least eighty percent the same as the hinge region of an antibody heavy chain;
(c) a third DNA sequence joined through a linking group in reading frame at its 5' end to the 3' end of said second DNA sequence, said third sequence coding for a polypeptide at least eighty percent the same as the C_{H}2 region of an antibody heavy chain; and
(d) a fourth DNA sequence joined through a linking group in reading frame at its 5' end to the 3' end of said third DNA sequence, said fourth sequence coding for a polypeptide at least eighty percent the same as the C_{H}3 region of an antibody heavy chain;
wherein at least one of said linking groups contains a restriction site unique to said construct.

14. The DNA construct of Claim 13, wherein two of said linking groups comprise unique restriction sites.

15. The DNA construct of Claim 13, additionally comprising a fifth DNA sequence joined through a linking group at its 5' end to the 3' end of said fourth sequence coding for a polypeptide at least eighty percent the same as the C_{H}4 region of an antibody heavy chain.

16. The DNA construct of Claim 13, wherein said linking group joining the sequence encoding the C-terminal domain of the antibody heavy chain to the preceding sequence comprises a termination codon or a non-sense codon.

17. A DNA construct for expression of a domain-modified constant region antibody heavy chain comprising:
(a) a first DNA sequence coding for a polypeptide at least eighty percent the same as the variable region of an antibody heavy chain;
(b) a second DNA sequence joined through a linking group in reading frame at its 5' end to the 3' end of said first DNA sequence, said second sequence coding for a polypeptide at least eighty percent the same as the C_{H}1, region of an antibody heavy chain;
(c) a third DNA sequence joined through a linking group in reading frame at its 5' end to the 3' end of said second DNA sequence, said third sequence coding for a polypeptide at least eighty percent the same as the hinge region of an antibody heavy chain;
(d) a fourth DNA sequence joined through a linking group in reading frame at its 5' end to the 3' end of said third DNA sequence, said fourth sequence coding for a polypeptide at least eighty percent the same as the C_{H}2 region of an antibody heavy chain; and
(e) a fifth DNA sequence joined through a linking group in reading frame at its 5' end to the 3' end of said fourth DNA sequence, said fifth sequence coding for a polypeptide at least eighty percent the same as the C_{H}3 region of an antibody heavy chain;
wherein said construct encodes a domain-modified antibody chain.

18. A DNA construct for expression of a domain-modified constant region antibody light chain comprising:
(a) a first DNA sequence coding for a polypeptide substantially the same as the variable region of an antibody light chain; and
(b) a second DNA sequence joined through a linking group in reading frame at its 5' end to the 3' end of said first DNA sequence, said second sequence coding for a polypeptide at least eighty percent the same as the C_{H}1 region of an antibody heavy chain.

19. A cell which contains DNA constructs of Claim 17 for expression of a domain-modified constant region antibody heavy chain.

20. A cell according to Claim 19 additionally comprising a DNA sequence encoding an antibody light chain.

21. A method for producing a domain-modified constant region antibody which comprises:
(a) growing cells according to Claim 20 in a nutrient medium, whereby said DNA constructs are expressed and said heavy and light chains are intracellularly bound together to form an antibody; and
(b) isolating said antibody.

## Patentansprüche

1. Antikörperkette mit zumindest einer Bindestellenregion und einer domänenmodifizierten Konstantregion, worin die domänenmodifizierte Konstantregion eine Modifikation umfaßt, die ausgewählt ist aus der Gruppe bestehend aus (1) einer Insertion von zumindest 80% der Aminosäuren zumindest einer der Domänen C_{L}, C_{H}1, Hinge, C_{H}2, C_{H}3 und C_{H}4, wobei der Rest der domänenmodifizierten Konstantregion die gleiche Aminosäuresequenz aufweist wie eine Konstantregion eines Säugetier-Antikörpers, (2) einer Substitution von zumindest 80% der Aminosäuren zumindest einer der Domänen C_{L}, C_{H}1, Hinge, C_{H}2, C_{H}3 oder C_{H}4 durch zumindest 80% der Aminosäuren zumindest einer, jedoch weniger als aller Domänen aus einer anderen Säugetier-Antikörperkette, wobei der Rest der domänenmodifizierten Konstantregion zu zumindest 80% die gleiche Aminosäuresequenz aufweist wie eine Konstantregion eines Säugetier-Antikörpers, und (3) einer Deletion von zumindest 80% der Aminosäuren mehr als einer der Domänen C_{H}1, Hinge, C_{H}2, C_{H}3 oder C_{H}4, worin die restlichen Aminosäuren der domänenmodifizierten Konstantregion zu zumindest 80% die gleiche Aminosäuresequenz aufweisen wie zumindest eine Domäne einer Konstantregion eines Säugetier-Antikörpers.

2. Antikörperkette nach Anspruch 1, worin die Bindestellenregion und die domänenmodifizierte Konstantregion aus der gleichen Säugetierquelle stammen.

3. Antikörperkette nach Anspruch 1, worin die Bindestellenregion aus einer ersten Säugetierquelle stammt und die domänenmodifizierte Konstantregion aus einer zweiten Säugetierquelle stammt.

4. Antikörperkette nach Anspruch 3, worin die erste Säugetierquelle der gleichen Spezies angehört wie die zweite Säugetierquelle.

5. Antikörperkette nach Anspruch 3, worin die erste Säugetierquelle einer anderen Spezies angehört als die zweite Säugetierquelle.

6. Antikörperkette nach Anspruch 1, worin die domänenmodifizierte Konstantregion eine Substitution einer korrespondierenden Domäne eines Antikörpers eines anderen Isotyps oder einer anderen Klasse umfaßt.

7. Antikörperkette nach Anspruch 1, worin die substituierte Domäne aus der gleichen Tierspezies stammt wie die Konstantregion.

8. Antikörperkette nach Anspruch 1, worin die Quelle der substituierten Domäne aus einer anderen Spezies stammt als die Quelle der Konstantregion.

9. Antikörperkette nach Anspruch 1, worin die Insertion zumindest zwei Domänen umfaßt.

10. Antikörperkette nach Anspruch 1, worin die Insertion die Verdopplung zumindest einer Domäne der Konstantregion ist.

11. Antikörper, umfassend zumindest eine Antikörperkette nach Anspruch 1.

12. DNA-Konstrukt, umfassend eine für eine Antikörperkette nach Anspruch 1 kodierende DNA-Sequenz.

13. DNA-Konstrukt zur Herstellung einer domänenmodifizierten Konstantregion einer schweren Kette eines Antikörpers, umfassend:
(a) eine erste DNA-Sequenz, die für ein Polypeptid kodiert, das zu zumindest 80% gleich ist der C_{H}1-Region einer schweren Kette eines Antikörpers;
(b) eine zweite DNA-Sequenz, die über eine Verbindungsgruppe im Leserahmen an ihrem 5'-Ende mit dem 3'-Ende der ersten DNA-Sequenz verbunden ist, wobei die zweite Sequenz für ein Polypeptid kodiert, das zu zumindest 80% gleich ist der Hinge-Region einer schweren Kette eines Antikörpers;
(c) eine dritte DNA-Sequenz, die durch eine Verbindungsgruppe im Leserahmen an ihrem 5'-Ende mit dem 3'-Ende der zweiten DNA-Sequenz verbunden ist, wobei die dritte Sequenz für ein Polypeptid kodiert, das zu zumindest 80% gleich ist der C_{H}2-Region einer schweren Kette eines Antikörpers; und
(d) eine vierte DNA-Sequenz, die über eine Verbindungsgruppe im Leserahmen an ihrem 5'-Ende mit dem 3'-Ende der dritten DNA-Sequenz verbunden ist, wobei die vierte Sequenz für ein Polypeptid kodiert, das zu zumindest 80% gleich ist der C_{H}3-Region einer schweren Kette eines Antikörpers;
worin zumindest eine der Verbindungsgruppen eine Restriktionsstelle enthält, die im Konstrukt jeweils einzigartig ist;

14. DNA-Konstrukt nach Anspruch 13, worin zwei der Verbindungsgruppen jeweils einzigartige Restriktionsstellen umfassen.

15. DNA-Konstrukt nach Anspruch 13, zusätzlich umfassend eine fünfte DNA-Sequenz, die über eine Verbindungsgruppe an ihrem 5'-Ende mit dem 3'-Ende der vierten Sequenz verbunden ist, die für ein Polypeptid kodiert, das zu zumindest 80% gleich ist der C_{H}4-Region einer schweren Kette eines Antikörpers.

16. DNA-Konstrukt nach Anspruch 13, worin die Verbindungsgruppe, die die Sequenz, die für die C-terminale Domäne der schweren Kette eines Antikörpers kodiert, mit der vorhergehenden Sequenz verbindet, ein Terminationscodon oder ein Nichtsense-Codon umfaßt.

17. DNA-Konstrukt zur Expresson einer schweren Kette eines Antikörpers mit domänenmodifizierter Konstantregion, umfassend:
(a) eine erste DNA-Sequenz, die für ein Polypeptid kodiert, das zu zumindest 80% gleich ist der variablen Region einer schweren Kette eines Antikörpers;
(b) eine zweite DNA-Sequenz, die über eine Verbindungsgruppe im Leserahmen an ihrem 5'-Ende mit dem 3'-Ende der ersten DNA-Sequenz verbunden ist, wobei die zweite Sequenz für ein Polypeptid kodiert, das zu zumindest 80% gleich ist der C_{H}1-Region einer schweren Kette eines Antikörpers;
(c) eine dritte DNA-Sequenz, die über eine Verbindungsgruppe im Leserahmen an ihrem 5'-Ende mit dem 3'-Ende der zweiten DNA-Sequenz verbunden ist, wobei die dritte Sequenz für ein Polypeptid kodiert, das zu zumindest 80% gleich ist der Hinge-Region einer schweren Kette eines Antikörpers;
(d) eine vierte DNA-Sequenz, die über eine Verbindungsgruppe im Leserahmen an ihrem 5'-Ende mit dem 3'-Ende der dritten DNA-Sequenz verbunden ist, wobei die vierte Sequenz für ein Polypeptid kodiert, das zu zumindest 80% gleich ist der C_{H}2-Region einer schweren Kette eines Antikörpers;
(e) eine fünfte DNA-Sequenz, die über eine Verbindungsgruppe im Leserahmen an ihrem 5'-Ende mit dem 3'-Ende der vierten DNA-Sequenz verbunden ist, wobei die fünfte Sequenz für ein Polypeptid kodiert, das zu zumindest 80% gleich ist der C_{H}3-Region einer schweren Kette eines Antikörpers;
worin das Konstrukt für eine domänenmodifizierte Antikörperkette kodiert.

18. DNA-Konstrukt zur Expression einer leichten Kette eines Antikörpers mit domänenmodifizierter Konstantregion, umfassend:
(a) eine erste DNA-Sequenz, die für ein Polypeptid kodiert, das im wesentlichen gleich ist der variablen Region einer leichten Kette eines Antikörpers; und
(b) eine zweite DNA-Sequenz, die über eine Verbindungsgruppe im Leserahmen an ihrem 5'-Ende mit dem 3'-Ende der ersten DNA-Sequenz verbunden ist, wobei die zweite Sequenz für ein Polypeptid kodiert, das zu zumindest 80% gleich ist der C_{H}1-Region einer schweren Kette eines Antikörpers.

19. Zelle, die DNA-Konstrukte nach Anspruch 17 zur Expression einer schweren Kette eines Antikörpers mit domänenmodifizierter Konstantregion enthält.

20. Zelle nach Anspruch 19, die zusätzlich eine DNA-Sequenz, die für eine leichte Kette eines Antikörpers kodiert, umfaßt.

21. Verfahren zum Herstellen eines Antikörpers mit domänenmodifizierter Konstantregion, umfassend:
(a) das Züchten von Zellen gemäß Anspruch 20 in einem Nährstoffmedium, wodurch die DNA-Konstrukte exprimiert und die schweren und leichten Ketten intrazellulär verbunden werden, um einen Antikörper zu bilden; und
(b) das Isolieren des Antikörpers.

## Revendications

1. Chaîne d'anticorps ayant au moins une région d'un site de liaison et une région constante à domaine modifié, où ladite région constante à domaine modifié comprend une modification sélectionnée dans le groupe consistant en (1) une insertion d'au moins quatre-vingts pour-cent des acides aminés d'au moins l'un des domaines de C_{L}, C_{H}1, charnière, C_{H}2, C_{H}3, et C_{H}4, ou le reste de la région constante à domaine modifié a la même séquence d'acides aminés que celle d'une région constante d'un anticorps mammalien, (2) une substitution d'au moins quatre-vingts pour-cent des acides aminés d'au moins l'un des domaines de C_{L}, C_{H}1, charnière, C_{H}3, ou C_{H}4 par au moins quatre-vingts pour-cent des acides aminés d'au moins l'un mais moins de tous lesdits domaines d'une chaîne d'un anticorps mammalien différent, où le reste de la région constante dont le domaine est modifié a au moins quatre-vingts pour-cent de la même séquence d'acides aminés qu'une région constante d'un anticorps mammalien et (3) une délétion d'au moins quatre-vingts pour-cent des acides aminés de plus que l'un des domaines C_{H}1, charnière, C_{H}2, C_{H}3, ou C_{H}4 où les acides aminés restants de la région constante dont le domaine est modifié ont au moins quatre-vingts pour-cent de la même séquence d'acides aminés qu'au moins un domaine d'une région constante d'un anticorps mammalien.

2. Chaîne d'anticorps de la revendication 1, où ladite région du site de liaison et ladite région constante dont le domaine est modifié proviennent de la même source mammalienne.

3. Chaîne d'anticorps de la revendication 1, où ladite région du site de liaison provient d'une première source mammalienne et ladite région constante dont le domaine est modifié provient d'une seconde source mammalienne.

4. Chaîne d'anticorps de la revendication 3, où ladite première source mammalienne est de la même espèce que ladite seconde source mammalienne.

5. Chaîne d'anticorps de la revendication 3, où ladite première source mammalienne est d'une espèce différente de ladite seconde source mammalienne.

6. Chaîne d'anticorps de la revendication 1, où ladite région constante dont le domaine est modifié comprend une substitution d'un domaine correspondant d'un anticorps d'un autre isotype de classe.

7. Chaîne d'anticorps de la revendication 1, où ledit domaine substitué est de la même espèce d'animal que ladite région constante.

8. Chaîne d'anticorps de la revendication 1, où la source dudit domaine substitué provient d'une espèce différente de la source de ladite région constante.

9. Chaîne d'anticorps de la revendication 1, où ladite insertion est d'au moins deux domaines.

10. Chaîne d'anticorps de la revendication 1, où ladite insertion est la duplication d'au moins un domaine de ladite région constante.

11. Anticorps comprenant au moins une chaîne d'anticorps de la revendication 1.

12. Construction d'ADN comprenant une séquence d'ADN codent pour une chaîne d'anticorps de la revendication 1.

13. Construction d'ADN pour produire une région constante dont le domaine est modifié d'une chaîne lourde d'anticorps comprenant:
(a) une première séquence d'ADN codant pour un polypeptide qui est au moins à quatre-vingts pour-cent le même que la région C_{H}1 d'une chaîne lourde d'anticorps;
(b) une seconde séquence d'ADN jointe par un groupe d'enchaînement en cadre de lecture à son extrémité côté 5' à l'extrémité côté 3' de ladite première séquence d'ADN, ladite seconde séquence codant pour un polypeptide qui est au moins à quatre-vingts pour-cent le même que la région de charnière d'une chaîne lourde d'anticorps;
(c) une troisième séquence d'ADN jointe par un groupe d'enchaînement au cadre de lecture à son extrémité côté 5' à l'extrémité côté 3' de ladite seconde séquence d'ADN, ladite troisième séquence codant pour un polypeptide qui est au moins à quatre-vingts pour-cent le même que la région C_{H}2 d'une chaîne lourde d'anticorps; et
(d) une quatrième séquence d'ADN jointe par un groupe d'enchaînement en cadre de lecture à son extrémité côté 5' à l'extrémité côté 3' de ladite troisième séquence d'ADN, ladite quatrième séquence codant pour un polypeptide qui est au moins à quatre-vingts pour-cent le même que la région C_{H}3 d'une chaîne lourde d'anticorps:
où au moins l'un desdits groupes d'enchaînement contient un site de restriction unique à ladite construction.

14. Construction d'ADN de la revendication 13, où deux desdits groupes d'enchaînement comprennent des sites uniques de restriction.

15. Construction d'ADN de la revendication 13, comprenant additionnellement une première séquence d'ADN jointe par un groupe d'enchaînement à son extrémité côté 5' à l'extrémité côté 3' de ladite quatrième séquence codant pour un polypeptide qui est au moins à quatre-vingts pour-cent le même que la régicn C_{H}4 d'une chaîne lourde d'anticorps.

16. Construction d'ADN de la revendication 13, où ledit groupe d'enchaînement joignant la séquence codant pour le domaine C-terminal de la chaîne lourde d'anticorps à la séquence précédente comprend un codon de terminaison ou un codon non-sens.

17. Construction d'ADN pour l'expression d'une chaîne lourde d'anticorps à région constante dont le domaine est modifié comprenant :
(a) une première séquence d'ADN codant pour un polypeptide qui est au moins à quatre-vingts pour-cent la même que la région variable d'une chaîne lourde d'anticorps;
(b) une seconde séquence d'ADN jointe par un groupe d'enchaînement en cadre de lecture à son extrémité coté 5' à l'extrémité côté 3' de ladite première séquence d'ADN, ladite seconde séquence codant pour un polypeptide qui est au moins à quatre-vingts pour-cent le même que la région C_{H}1 d'une chaîne lourde d'anticorps;
(c) une troisième séquence d'ADN jointe par un groupe d'enchaînement en cadre de lecture à son extrémité côté 5' à l'extrémité côté 3' de ladite seconde séquence d'ADN, ladite troisième séquence codant pour un polypeptide qui est au moins à quatre-vingts pour-cent le même que la région de charnière d'une chaîne lourde d'anticorps;
(d) une quatrième séquence d'ADN jointe par un groupe d'enchaînement au cadre de lecture à son extrémité côté 5' à l'extrémité côté 3' de ladite troisième séquence d'ADN, ladite quatrième séquence codent pour un polypeptide qui est au moins à quatre-vingts pour-cent le même que la région C_{H}2 d'une chaîne lourde d'anticorps:
(e) une cinquième séquence d'ADN jointe par un groupe d'enchaînement en cadre de lecture à son extrémité côté 5' à l'extrémité côté 3' de ladite quatrième séquence d'ADN, ladite cinquième séquence codant pour un polypeptide qui est au moins à quatre-vingts pour-cent le même que la région C_{H}3 d'une chaîne lourde d'anticorps:
où ladite construction code pour une chaine d'anticorps dont le domaine est modifié.

18. Construction d'ADN pour l'expression d'une chaîne légère d'anticorps à région constante dont le domaine est modifié comprenant :
(a) une première séquence d'ADN codant pour un polypeptide sensiblement identique à la région variable d'une chaîne légère d'anticorps; et
(b) une seconde séquence d'ADN jointe par un groupe d'enchaînement en cadre de lecture à son extrémité côté 5' à l'extrémité côté 3' de ladite première séquence d'ADN, ladite seconde séquence codant pour un polypeptide qui est au moins à quatre-vingts pour-cent le même que la région C_{H}1 d'une chaîne lourde d'anticorps.

19. Cellule qui contient des constructions d'ADN de la revendication 17, pour l'expression d'une chaîne lourde d'anticorps à région constante dont le domaine est modifié.

20. Cellule selon la revendication 19, comprenant additionnellement une séquence d'ADN codant pour une chaîne légère d'anticorps.

21. Méthode de production d'un anticorps à région constante dont le domaine est modifié qui comprend :
(a) la croissance de cellules selon la revendication 20 dans un milieu nutritif, ce par quoi lesdites constructions d'ADN sont exprimées et lesdites chaînes lourde et légère sont intracellulairement liées ensemble pour former un anticorps; et
(b) l'isolement dudit anticorps.
